# EUROPEAN PATENT SPECIFICATION

(11) **EP 0 679 096 B1**
(45) Date of publication and mention of the grant of the patent: **08.12.1999**
(21) Application number: 94904268.3
(22) Date of filing: 17.01.1994
(51) Int. Cl.: A61L 27/00

(54) **LOAD BEARING IMPLANTABLE PROSTHESIS**
LASTTRAGENDE IMPLANTIERBARE PROTHESE
PROTHESE IMPLANTABLE SUPPORT DE CHARGE

(30) Priority: 15.01.1993 GB 9300746
(43) Date of publication of application: 02.11.1995
(73) Proprietor: FINSBURY (INSTRUMENTS) LIMITED, Learherhead, Surrey KT22 OBA (GB)
(72) Inventor: EVANS, Samuel Lewin, Hants SO2 0PR (GB); GREGSON, Peter John, Hants SO51 7JZ (GB)
(74) Representative: Pennant, Pyers
(86) International application number: GB9400085
(87) International publication number: WO9415652

(56) References cited:
- EP-A- 0 159 036
- EP-A- 0 340 174
- DE-A- 4 126 800
- US-A- 4 202 055

## Description

This invention is concerned with a prosthesis having a desirable combination of biological, chemical and mechanical properties. The invention is particularly concerned with load-bearing structural implantable prostheses for humans or mammals, e.g. for orthopaedic or dental or maxillofacial purposes. An example of such a load-bearing orthopaedic implantable prosthesis is a hip joint.

U.S. Patent 4,202,055 (Battelle) describes a hip joint prosthesis in which the anchorage has a non-porous outer coating comprising calcium phosphate particles embedded in an epoxy adhesive. The calcium phosphate particles dissolve in vivo, creating an open-pore continuous network for ingrowth of bone. The problem which arises from the generally differing mechanical characteristics of the core of the prosthesis, in comparison with the surrounding bone tissue, is not addressed.

In load bearing prostheses, such as for example replacement hip joints, the mechanical characteristics are substantially different from those of the bone which the prosthesis replaces, and this leads to an unnatural stress distribution in the vicinity of the implant. R. Shirandami and I. I. Esat describe (Journal of Biomedical Engineering, 1990, Vol. 12, January, pages 19-22) a prosthesis having a layered structure in which the outer layer has a lower tensile modulus (stiffness) than the core. The treatment is mathematical, and the authors did not address the effect of the lower modulus layer on the stresses across the interface between the implant and host tissue. Furthermore the biological and chemical properties of the prosthesis are not discussed.

There have been difficulties in bonding bioactive coatings to metallic prosthesis cores. Plasma spraying has been used, but the resulting ceramic layer may be chemically degraded and is poorly bonded to the substrate.

L. M. Boulton et al. describe (Materials Letters 12, 1991, pages 1-6) an adhesively bonded hydroxyapatite coating, using a single part toughened epoxy adhesive, and hydroxyapatite granules embedded uniformly within the adhesive. After curing the adhesive, loose hydroxyapatite granules were removed from the surface using a high pressure water jet. Improved stress distribution, when compared with plasma-sprayed hydroxyapatite, was demonstrated.

EP 0 413 492 describes a prosthetic implant which is at least partially coated with demineralized bone powder.

EP-A-0 340 174 relates to a composite-material prosthesis femoral stem, comprising a plurality of layers made of Kevlar® or other like materials impregnated and/or coated with different particle size powders of alumina or zirconium oxydes or hydroxylapatite

US 5,047,054 describes prostheses in which the core is coated to provide a seal for the substrate. The effect of the coating on the mechanical performance of the implant is not mentioned.

In one aspect this invention provides prosthesis comprising a load-bearing core and, bonded on or integral with a surface thereof, an interlayer of an organic polymeric material which interlayer has a shear modulus below that of the load-bearing core, and an outer layer of particles of biocompatible material, the particles being adhesively bonded at the outer surface of the interlayer.

In another aspect, the invention provides a method of making a prosthesis as herein described, which method comprises providing a load-bearing core, applying to a surface thereof an organic polymeric material to form an interlayer thereon, and applying to the interlayer particles of a biocompatible material under conditions to cause the particles to be adhesively bonded at the surface of the interlayer.

The nature of the load-bearing core is not material to the invention. Suitable for many purposes are metallic cores based on Co-Cr-Mo alloys, titanium alloys and stainless steel. Also suitable are polymeric materials, which may be unreinforced or reinforced e.g. with carbon or other fibres. Polymers which have been used include acetal, epoxy, polyether ether ketone, polyether sulphone, acrylic, and UHMW polyethylene. The core needs to have strength and stiffness properties sufficient to withstand the stresses to be encountered in vivo.

Bonded on or integral with a surface of the core is an interlayer of an organic polymeric material. This interlayer extends over the whole, or more usually over a part, of the surface of the core. The interlayer performs several functions:
- It has a lower shear modulus than the core, and as a result acts to spread the load when the prosthesis is under stress in vivo.
- It provides chemical protection for the surrounding tissues from the core.
- It acts as an interlayer onto which the surface layer is bonded.

The shear modulus of the interlayer is preferably 1-30 GPa, particularly 2-10 GPa.

The interlayer is preferably 0.1 to 10 mm, more particularly 0.2 to 1.0 mm, thick. Below these ranges, the interlayer may not be effective to spread the load and reduce damage to surrounding bone. Thicker layers provide no added advantage, and merely reduce the overall mechanical properties of the prosthesis. In a hip joint prosthesis, the thickness of the interlayer nay vary from the proximal to the distal regions.

The organic polymeric material of the interlayer may be any biocompatible thermoplastic or thermosetting polymer with adhesive properties. Suitable are those polymers noted above for use in the core. Toughened epoxy resins are preferred. The polymer may be applied to the core as a fluid coating. A viscosity adjuster, such as fumed silica, or other fine particulate material, may be included. A biocompatible filler or reinforcement may also be provided, e.g. 1 to 50% by weight of hydroxyapatite particles having an average size in the range 0.1 to 20 µm.

The outer layer of the prosthesis of this invention comprises particles of biocompatible material, preferably but not necessarily inorganic, and an adhesive which bonds the particles to the interlayer. The particles are preferably 20 µm up to 2 mm, particularly 100 µm up to 1.0 mm, in diameter. The particles may be bioinert ceramics such as alumina, but are preferably bioactive, meaning that they interact with living bone tissue. Preferred are calcium phosphate particles e.g. hydroxyapatite, whose chemical composition may vary depending on the CaO:P₂0₅ ratio, or other bioactive materials e.g. fluoroapatite. These particles are preferably sintered and slightly porous. They may be coated with an organosilane, to improve their adhesion to the adhesive. These particles are adhesively bonded to the outer surface of the interlayer, preferably by being at least partly embedded in the adhesive of the outer layer. The outer layer may not entirely overlie the interlayer.

The adhesive of the outer layer may be an organic polymeric material, the same as or different to that of the interlayer. A toughened epoxy resin is preferred.

The outer layer thus comprises the particles of inorganic material and the adhesive in which they are bonded. Preferably the inorganic particles comprise at least 30% of the surface. Other bioactive materials, such as for example suitable proteins and antibiotics may be incorporated in the outer layer.

As described in the example below, we prefer to lay down the coating as two separate layers. The first applied or interlayer is a toughened one-part epoxy adhesive, which is unfilled but may contain a viscosity adjuster. The thickness of this layer is varied to provide the desired mechanical properties for the prosthesis. The second or outer layer comprises a toughened one-part epoxy adhesive which is filled with relatively fine (e.g. 1 µm) particles of a biocompatible material such as hydroxyapatite. The thickness of this outer layer is sufficient merely to bind and embed the subsequently applied larger particles.

When the load-bearing core is of metal, the interlayer overlies and is adhesively bonded to a surface of the core, which may be profiled or otherwise prepared for this purpose. When the core is of a polymeric material, then the transition from core to interlayer may be less clear-cut. The interlayer may be formed of the same organic polymeric material as the core. The desired differing shear modulus and other mechanical properties may be achieved by providing different levels of filler or reinforcement in the interlayer and in the core. The core and interlayer may be designed together to have functionally graded properties.

This invention also provides a method of making a prosthesis, which prosthesis is preferably but not necessarily a prosthesis as described above. The method involves applying to a surface of a load-bearing prosthesis core an organic polymeric adhesive to form an interlayer of desired thickness thereon. This may then be partially or fully cured; or its viscosity may be arranged to be so high that the interlayer is not significantly penetrated by the subsequently applied bioactive material particles. Then particles of a biocompatible material are adhesively bonded to the outer surface of this interlayer. Slightly porous hydroxyapatite particles are preferred, but other materials such as fluoroapatite or tricalcium phosphate are possible alternatives. The particles may be sprinkled on to the adhesive of the outer layer or applied in a stream of air, or the prosthesis carrying the adhesive layer may be dipped in a bed of the particles.

The adhesive of the outer layer (and perhaps also the interlayer) is then cured, e.g. by heating. During curing, the adhesive penetrates the pores of the particles, thus reinforcing them.

Superficial particles are then removed, preferably together with the outer part of the base layer of particles and any adhesive which may be covering them. This removal may be effected by any appropriate mechanical treatment, but is preferably performed by bead blasting. This step results in a layer of strongly bonded half-particles with a large area of exposed hydroxyapatite or other bioactive material. These particles are reinforced by the adhesive which has penetrated into them during curing.

The following example illustrates the method and prosthesis of the invention.

### EXAMPLES

### EXAMPLE 1

A hip joint prosthesis of the Freeman design with a Ti-Al-V stem was used for this experiment. The stem had a bead blasted surface. To regions around the stem were applied two layers to form together a coating:
a) The first layer (the interlayer) was of a one-part toughened epoxy heat curable resin sold by Permabond under the reference ESPP770. The adhesive contained 3.5% by weight of Cab-o-Sil fumed silica as a thickener. The resin was applied by spray or dipping to form a layer about 100 µm thick around the proximal part of the stem. The device was heated to 160°C for 20 minutes and allowed to cool.
b) The same epoxy resin was used, but the 3.5% by weight of fumed silica was replaced by 35% by weight of hydroxyapatite powder of 1 µm average diameter. This adhesive was applied by spraying or dipping to form a layer approximately 150 µm thick over the adhesive a).

While this surface layer was still fluid, a supply of hydroxyapatite particles of average size 250 µm was poured over it. A monolayer of the particles adhered to the resin and sank into it as a result of surface tension effects, forming a layer at the surface of the interlayer. The prosthesis was then heated at 160°C for 70 minutes to cure the epoxy resin.

The coated prosthesis was then bead blasted with 100 to 150 µm glass beads at 140 kPa for 30 seconds. This had the effect of taking off the tops of the hydroxyapatite surface particles, together with any overlying adhesive, so that the remaining outer surface was substantially formed of hydroxyapatite.

The following Examples 2, 3, 4, 5 and 6 demonstrate the improved properties of implant materials coated according to the invention, over conventional plasma sprayed coatings. In each case, coatings were applied in the manner described in Example 1. In some cases, comparative test specimens were coated by plasma spray coating.

### Example 2

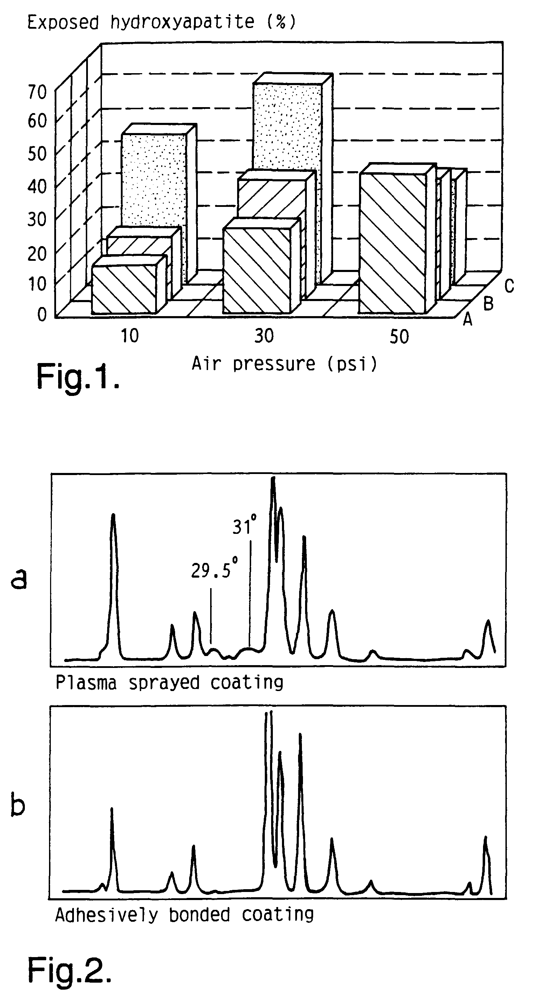

Coatings applied to implant materials in the manner described in Example 1 were examined to assess the exposed hydroxyapatite after bead blasting. As shown in Figure 1, a substantial area of exposed hydroxyapatite was produced for a range of air pressures used.
- A: represents bead blasting for 10 sec at 15° to the surface.
- B: represents bead blasting for 10 sec at 90° to the surface.
- C: represents bead blasting for 60 sec at 90° to the surface.
At an air pressure of 50 psi, all conditions produced a desired hydroxyapatite exposure of 30 - 50%. Without bead blasting the hydroxyapatite exposure was close to 0%.

### Example 3

X-ray diffraction results for an adhesively bonded coating according to the invention and for a conventional plasma sprayed coating are shown in Figures 2a and b respectively. The adhesive bonding process avoids the detrimental microstructural changes that result from plasma spraying, and which are visible at 29.5° and 31°.

### Example 4

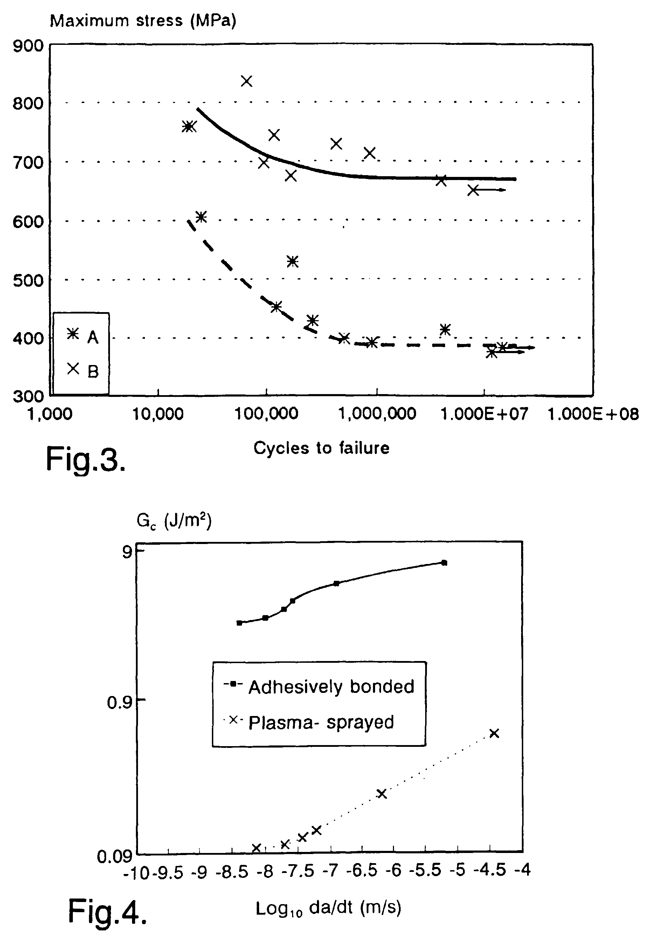

Rolls-Royce rotating bend fatigue tests were carried out in flowing, aerated Ringer's solution. Hourglass-shaped specimens of annealed Ti-6Al-4V alloy were coated with a conventional plasma-sprayed hydroxyapatite coating or with an adhesively bonded coating. Figure 3 shows S-N curves for each condition and clearly illustrates the improved performance of the adhesively coated specimens. In Figure 3:
- A.: Conventional plasma sprayed coating;
- B.: Adhesively bonded coating.
Uncoated specimens performed the same as B. It is apparent that plasma spraying has damaged the substrate, viz the lower curve A.

### Example 5

A modified Boeing wedge cleavage test was carried out on adhesively bonded coatings to Ti-6Al-4V alloy, and on plasma-sprayed coatings. The tests were carried out in Ringer's solution at 37°C. Figure 4 shows the critical strain energy release rate G_{c} as a function of the slow crack growth rate da/dt. The adhesively bonded coating has a greater strength of adhesion.

### Example 6

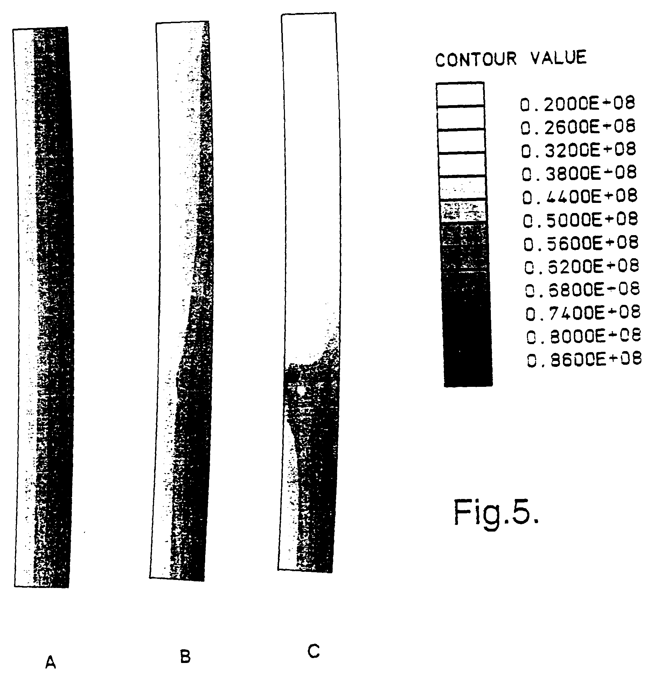

Axisymmetric finite element analysis was performed on the proximal femur. Figure 5 shows stress distribution in the femur, around the distal tip of a hip prosthesis in:
- A.: Natural femur;
- B.: Prosthesis according to the invention, with adhesively bonded coating;
- C.: Conventional plasma sprayed prosthesis.

In B and C, a metal implant has been inserted to approximately two thirds of the way down the femur as shown. A downwards axial load of 5kN was applied and a bending movement to the left of 150 Nm was applied at the top.

The more natural stress distribution around the adhesively bonded coated prosthesis is evident.

## Claims

1. A prosthesis comprising a load-bearing core and, bonded on or integral with a surface thereof, an interlayer of an organic polymeric material which interlayer has a shear modulus below that of the load-bearing core, and an outer layer of particles of a biocompatible material, the particles being adhesively bonded at the outer surface of the interlayer.

2. A prosthesis as claimed in claim 1, wherein the interlayer is of an epoxy resin.

3. A prosthesis as claimed in claim 1 or claim 2, wherein the interlayer is 0.1 - 10 mm thick.

4. A prosthesis as claimed in any one of claims 1 to 3, wherein the biocompatible material is a bioactive material based on a calcium phosphate.

5. A prosthesis as claimed in any one of claims 1 to 4, wherein the particles are from 20 µm to 2 mm in diameter.

6. A prosthesis claimed in any one of claims 1 to 5, wherein the particles are porous.

7. A prosthesis as claimed in any one of claims 1 to 6, which is for an orthopaedic prosthesis.

8. A method of making a prosthesis as claimed in any one of claims 1 to 7, which method comprises providing a load-bearing core, applying to a surface thereof an organic polymeric material to form an interlayer thereon, applying to the interlayer a surface of particles of a biocompatible material under conditions to cause the particles to be adhesively bonded at the surface of the interlayer.

9. A method as claimed in claim 8, wherein, after curing the adhesive, the prosthesis is bead-blasted to remove parts of the inorganic particles on the surface.

## Patentansprüche

1. Prothese, umfassend einen lasttragenden Kern und, verbunden oder als Einheit mit einer Oberfläche desselben, eine Zwischenschicht aus einem organischen Polymermaterial, welche Zwischenschicht ein Schermodul unter dem des lasttragenden Kerns besitzt, sowie eine äußere Schicht von Partikeln aus einem biokompatiblen Material, wobei die Partikel klebend an die äußere Oberfläche der Zwischenschicht gebunden sind.

2. Prothese nach Anspruch 1, bei der die Zwischenschicht aus einem Epoxidharz ist.

3. Prothese nach Anspruch 1 oder Anspruch 2, bei der die Zwischenschicht 0,1 ∼ 10 mm dick ist.

4. Prothese nach einem der Ansprüche 1 bis 3, bei der das biokompatible Material ein bioaktives Material basierend auf einem Calciumphosphat ist.

5. Prothese nach einem der Ansprüche 1 bis 4, bei der die Partikel einen Durchmesser von 20 µm bis 2 mm besitzen.

6. Prothese nach einem der Ansprüche 1 bis 5, bei der die Partikel porös sind.

7. Prothese nach einem der Ansprüche 1 bis 6, die für eine orthopädische Prothese ist.

8. Verfahren zur Herstellung einer Prothese nach einem der Ansprüche 1 bis 7, welches Verfahren umfasst: Bereitstellen eines lasttragenden Kerns, Aufbringen eines organischen Polymermaterials auf eine Oberfläche desselben, um darauf eine Zwischenschicht Zu bilden, Aufbringen einer Oberfläche aus Partikeln eines biokompatiblen Materials auf die Zwischenschicht unter Bedingungen, die bewirken, dass die Partikel klebend an die Oberfläche der Zwischenschicht gebunden werden.

9. Verfahren nach Anspruch 8, bei dem nach Aushärten des Klebers die Prothese körnergestrahlt wird, um Teile der anorganischen Partikel auf der Oberfläche zu entfernen.

## Revendications

1. Prothèse comportant un noyau de support de charge et, fixée sur une surface de celui-ci ou dans le même bloc que celui-ci, une couche intermédiaire constituée d'un matériau de polymère organique, laquelle couche intermédiaire a un module de cisaillement situé en dessous de celui du noyau de support de charge, et une couche extérieure de particules de matériau biocompatible, les particules étant fixées de manière adhésive à la surface extérieure de la couche intermédiaire.

2. Prothèse selon la revendication 1, dans laquelle la couche intermédiaire est constituée d'une résine époxy.

3. Prothèse selon la revendication 1 ou 2, dans laquelle la couche intermédiaire a une épaisseur de 0,1 à 10 mm.

4. Prothèse selon l'une quelconque des revendications 1 à 3, dans laquelle le matériau biocompatible est un matériau bioactif à base de phosphate de calcium.

5. Prothèse selon l'une quelconque des revendications 1 à 4, dans laquelle les particules ont un diamètre allant de 20 µm à 2 mm.

6. Prothèse selon l'une quelconque des revendications 1 à 5, dans laquelle les particules sont poreuses.

7. Prothèse selon l'une quelconque des revendications 1 à 6, qui est destinée à une prothèse orthopédique.

8. Procédé de fabrication d'une prothèse selon l'une quelconque des revendications 1 à 7, lequel procédé consiste à fournir un noyau de support de charge, à appliquer sur une surface de celui-ci un matériau de polymère organique pour former une couche intermédiaire sur celui-ci, à appliquer sur la couche intermédiaire une surface de particules de matériau biocompatible dans des conditions amenant les particules à être fixées de manière adhésive à la surface de la couche intermédiaire.

9. Procédé selon la revendication 8, dans lequel, après durcissement de l'adhésif, la prothèse est grenaillée pour supprimer des parties des particules inorganiques situées sur la surface.
